# EUROPEAN PATENT APPLICATION

(11) **EP 2 657 680 A1**
(43) Date of publication of application: **30.10.2013**
(21) Application number: 11851528.7
(22) Date of filing: 01.12.2011
(51) Int. Cl.: G01N 21/17, A61B 1/24

(54) **OPTICAL TOMOGRAPHY IMAGE ACQUISITION DEVICE**

(30) Priority: 21.12.2010 JP 2010283993; 21.12.2010 JP 2010283995; 09.02.2011 JP 2011025739
(71) Applicant: Panasonic Corporation, Osaka 571-8501 (JP)
(72) Inventor: IIO, Masateru, Osaka-shi, Osaka 540-6207 (JP); HIRAI, Yasushi, Osaka-shi, Osaka 540-6207 (JP); KAWATA, Hiroto, Osaka-shi, Osaka 540-6207 (JP)
(74) Representative: Eisenführ, Speiser & Partner
(86) International application number: PCT/JP2011/006738
(87) International publication number: WO 2012/086134

(57) **Abstract**

The optical tomography image acquisition device comprises a tomographic image computer (23) that produces tomographic image information about a measurement object, a measurement distance range deviation detector (43) that detects that the distance between a measurement object and the light input/output portion (2) of a probe is outside a measurement distance range on the basis of the computation result of the tomographic image computer (23), and a display controller (44) that outputs the computation result of the tomographic image computer (23) to a display component (17) and also sends the detection result of the measurement distance range deviation detector (43) to the display component (17).

## Description

### TECHNICAL FIELD

The present invention relates to an optical tomography image acquisition device that is used for medical treatment, for example.

### BACKGROUND ART

A conventional optical tomography image acquisition device used for medical treatment had the following configuration.

Specifically, a conventional optical tomography image acquisition device had a configuration comprising a light source, a splitter that split light emitted from the light source into at least one direction and another direction, a probe that directed one of the split light beams from a light input/output portion toward a measurement object and that took in reflected light from the measurement object as measurement light, a reference mirror that performed path correction on the other light beam split by the splitter, an interference section that produced interference light by causing interference between the light that underwent path correction at the reference mirror and the measurement light taken in by the probe, a tomographic image computer that produced tomographic image information about a measurement object by computational processing of interference light produced at the interference section, and a display controller that outputted the computation result of the tomographic image computer to a display component (see Patent Literature 1, for example).

### CITATION LIST

### PATENT LITERATURE

Patent Literature 1: International Laid-Open Patent Application WO2007/060973

### SUMMARY

A problem encountered with the above-mentioned conventional optical tomography image acquisition device was that an accurate optical tomography image could not be displayed.

Specifically, with the above-mentioned conventional optical tomography image acquisition device, a probe that directed light at a measurement object and took in light reflected from that measurement object as measurement light was provided in order to display tomographic image information about the measurement object. When this device was used for dentistry, for example, the user held the probe in his hand, and took measurements with the probe close to a tooth so that it would be easier to shine the probe at the tooth that was the measurement object.

Since the user could adjust the irradiation angle and irradiation position of light from the probe by hand, this made the probe more convenient to use, but a problem was that a tomographic image of the measurement object could not be accurately displayed.

The present inventors conducted diligent study into the cause of not being able to accurately acquire a tomographic image of a measurement object, and discovered that if the light input/output portion of the probe is moved too close, beyond the measurement limit for the tooth that is the measurement object, then aliasing will occur, in which measurement data that is outside the measurement range is displayed superposed over other measurement data. It was found that this is why a tomographic image of the measurement object could not be accurately displayed.

In view of this, it is an object of the present invention to prevent aliasing from occurring when the light input/output portion of a probe is moved too close to a measurement object, and thereby display an accurate tomographic image.

To achieve this object, the optical tomography image acquisition device pertaining to a first invention comprises a light source, a splitter, a probe, a reference mirror, an interference section, a tomographic image computer, a measurement distance range deviation detector, and a notification section. The splitter splits light emitted from the light source into a first direction and a second direction. The probe has a light input/output portion that directs first light split off in the first direction toward a measurement object, and takes in reflected light from the measurement object as measurement light. The reference mirror performs path correction on second light split off in the second direction at the splitter. The interference section produces interference light by causing interference between the second light that has undergone path correction at the reference mirror and the first light taken in as measurement light to the probe. The tomographic image computer produces tomographic image information about a measurement object by computational processing of interference light produced at the interference section. The measurement distance range deviation detector detects whether or not the distance between the measurement object and the light input/output portion of the probe is outside of a measurement distance range on the basis of the computation result of the tomographic image computer. The notification section notifies of the detection result by the measurement distance range deviation detector.

The optical tomography image acquisition device pertaining to a second invention comprises a light source, a splitter, a probe, a reference mirror, an interference section, a tomographic image computer, and a surface detection processor. The splitter splits light emitted from the light source into a first direction and a second direction. The probe has a light input/output portion that directs first light split off in the first direction toward a measurement object, and takes in reflected light from the measurement object as measurement light. The reference mirror performs path correction on second light split off in the second direction at the splitter. The interference section produces interference light by causing interference between the second light that has undergone path correction at the reference mirror and the first light taken in as measurement light to the probe. The tomographic image computer produces tomographic image information about a measurement object by computational processing of interference light produced at the interference section. The surface detection processor outputs the computation result of the tomographic image computer and has a binary processor, a contraction processor, an expansion processor, a surface detector, and a correction processor. The binary processor binarizes a brightness value for display image data obtained from the tomographic image computer into 0 and a normalized reference value with respect to a reference value. The contraction processor sets the brightness value for a specific pixel to 0 when the brightness value is 0 for at least one pixel out of the surrounding pixels of a specific pixel of binarized display image data. The expansion processor sets the brightness value of a specific pixel to a normalized reference value when the brightness value of at least one pixel out of the surrounding pixels of a specific pixel of display image data that has undergone contraction processing is a normalized reference value. The surface detector detects the surface of a measurement object from display image data that undergone expansion processing. The correction processor compares display image data obtained from the tomographic image computer and the surface of a measurement object detected by the surface detector and performs display image correction.

The optical tomography image acquisition device pertaining to the third invention comprises a light source, a splitter, a probe, a reference mirror, an interference section, a tomographic image computer, and an oral cavity insertion portion type determiner. The splitter splits light emitted from the light source into a first direction and a second direction. The probe has a probe main body, an optical scanner that directs first light split off in the first direction toward a measurement object while varying the irradiation direction and that is provided inside the probe main body, and an oral cavity insertion portion that is provided with a light input/output portion that takes in reflected light from the measurement object as measurement light and that is removably attached to the probe main body. The reference mirror performs path correction on second light split off in the second direction at the splitter. The interference section produces interference light by causing interference between the second light that has undergone path correction at the reference mirror and the first light taken in as measurement light to the probe. The tomographic image computer produces tomographic image information about a measurement object by computational processing of interference light produced at the interference section. The oral cavity insertion portion type determiner determines the type of oral cavity insertion portion on the basis of the computation result of the tomographic image computer.

### ADVANTAGEOUS EFFECTS

With the first invention, when light is directed at a measurement object, it is detected that the distance between the measurement object and the light input/output portion of the probe is too short and is outside the measurement distance range, a notification is sent to this effect, and the user can recognize from the current measurement data that the probe is outside the measurement range for the measurement object. Thus, the user can adjust the position of the probe so that the light input/output portion of the probe is located within the measurement range, which allows an accurate optical tomography image to be displayed.

With the second invention, the mistaken detection of the surface of a measurement object by the surface detection processor, which detects the surface of a measurement object, can be avoided, so a more accurate tomographic image can be displayed, and this makes the tomographic image display easier to view.

With the third invention, the optical scanner of the probe directs light at the main body of the oral cavity insertion portion, takes in the reflected light as measurement light, and can determine the type of oral cavity insertion portion on the basis of the computation result of the tomographic image computer for interference light obtained from this measurement light. Thus, a function with which the device is equipped can be used to determine the type of oral cavity insertion portion, and there is no need to provide any switches or other such electrical or mechanical detectors, which allows the probe to be more compact.

### BRIEF DESCRIPTION OF DRAWINGS

FIG. 1 is an oblique view of a usage example of the optical tomography image acquisition device pertaining to an embodiment of the present invention;
FIG. 2 is an oblique view of the optical tomography image acquisition device in FIG. 1;
FIG. 3 is a diagram of a display screen of the display component of the optical tomography image acquisition device in FIG. 2;
FIG. 4 is an exploded oblique view of the optical tomography image acquisition device in FIG. 2;
FIG. 5 is an exploded oblique view of the optical tomography image acquisition device in FIG. 2;
FIG. 6 is an exploded oblique view of the main components of the optical tomography image acquisition device in FIG. 2;
FIG. 7 is an electrical block diagram of the optical tomography image acquisition device in FIG. 2;
FIG. 8 is a cross section of the main components of the optical tomography image acquisition device in FIG. 2 when in use;
FIG. 9 is a diagram of a display screen of the display component when aliasing occurs with the optical tomography image acquisition device in FIG. 2;
FIGS. 10a to 10d are diagrams of the relation between the frequency spectrum of interference light and the display screen of the optical tomography image acquisition device in FIG. 2;
FIG. 11 is a flowchart of aliasing detection by the optical tomography image acquisition device in FIG. 2;
FIG. 12 is a diagram of a display screen of the display component when aliasing occurs with the optical tomography image acquisition device in FIG. 2;
FIG. 13 is an electrical block diagram of the optical tomography image acquisition device pertaining to another embodiment of the present invention;
FIG. 14 is a cross section of the main components of the optical tomography image acquisition device in FIG. 13 when in use;
FIG. 15 is a diagram of a display screen of the display component of the optical tomography image acquisition device in FIG. 13;
FIG. 16 is a graph of the frequency spectrum of interference light with the optical tomography image acquisition device in FIG. 13;
FIG. 17 is a diagram of a display screen of the display component of the optical tomography image acquisition device in FIG. 13;
FIG. 18 is a diagram of the pixel layout in the display component of the optical tomography image acquisition device in FIG. 13;
FIG. 19 is a diagram of a display screen of the display component of the optical tomography image acquisition device in FIG. 13;
FIG. 20 is a diagram of a display screen of the display component of the optical tomography image acquisition device in FIG. 13;
FIG. 21 is a diagram of the pixel layout in the display component of the optical tomography image acquisition device in FIG. 13;
FIG. 22 is a diagram of a display screen of the display component of the optical tomography image acquisition device in FIG. 13;
FIG. 23 is a diagram of a display screen of the display component of the optical tomography image acquisition device in FIG. 13;
FIG. 24 is an electrical block diagram of the main components of the optical tomography image acquisition device in FIG. 13;
FIG. 25 is a flowchart of the flow up to the display of an optical tomographic image by the optical tomography image acquisition device in FIG. 13;
FIG. 26 is an electrical block diagram of the optical tomography image acquisition device pertaining to yet another embodiment of the present invention;
FIG. 27 is a cross section of the main components of the optical tomography image acquisition device in FIG. 26 when in use;
FIG. 28 is an exploded oblique view of the main components of the optical tomography image acquisition device in FIG. 26;
FIG. 29 is a cross section of the main components of the optical tomography image acquisition device in FIG. 26 when in use;
FIG. 30 is a graph of the frequency spectrum of interference light with the optical tomography image acquisition device in FIG. 26; and
FIG. 31 is a flowchart of determination of the type of oral cavity insertion portion by the optical tomography image acquisition device in FIG. 26.

### DESCRIPTION OF EMBODIMENTS

An embodiment of the present invention will be described through reference to the appended drawings.

In the following description, the "front and back direction" shall mean a direction corresponding to the lengthwise direction of the probe of the optical tomography image acquisition device. The "front" shall mean the distal end side that is inserted into the oral cavity, while the "rear" shall mean the opposite side.

### Embodiment 1

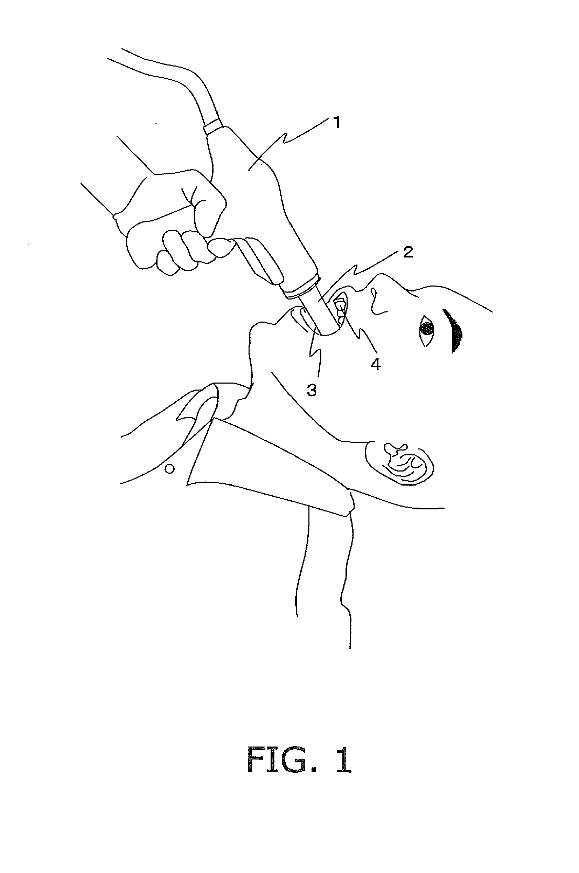

FIG. 1 shows a usage example of the optical tomography image acquisition device pertaining to this embodiment. As shown in FIG. 1, a light input/output portion (oral cavity insertion portion) 2 is mounted in a protruding state in front of a probe main body 1.

Although this will be described in detail at a later point, with the optical tomography image acquisition device pertaining to this embodiment, as shown in FIG. 1, the light input/output portion 2 is inserted into an oral cavity 3, and an optical tomographic image in the X axis direction of a tooth 4 is continuously acquired in the Y axis direction.

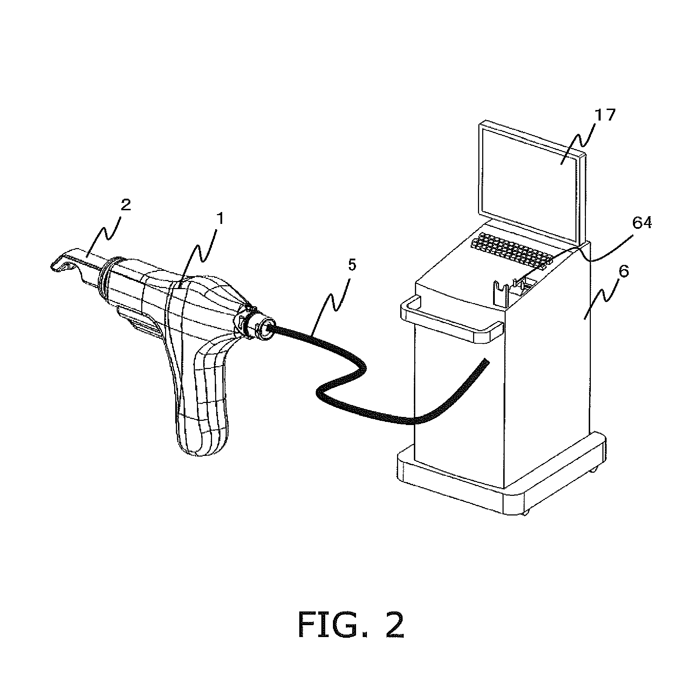

As shown in FIG. 2, the probe main body 1 is in the form of a pistol so that it can be held in one hand. A control box 6 is connected via a cable 5 to the rear end of the probe main body 1. Wiring for the input and output of light and for electrical signals is housed inside the cable 5.

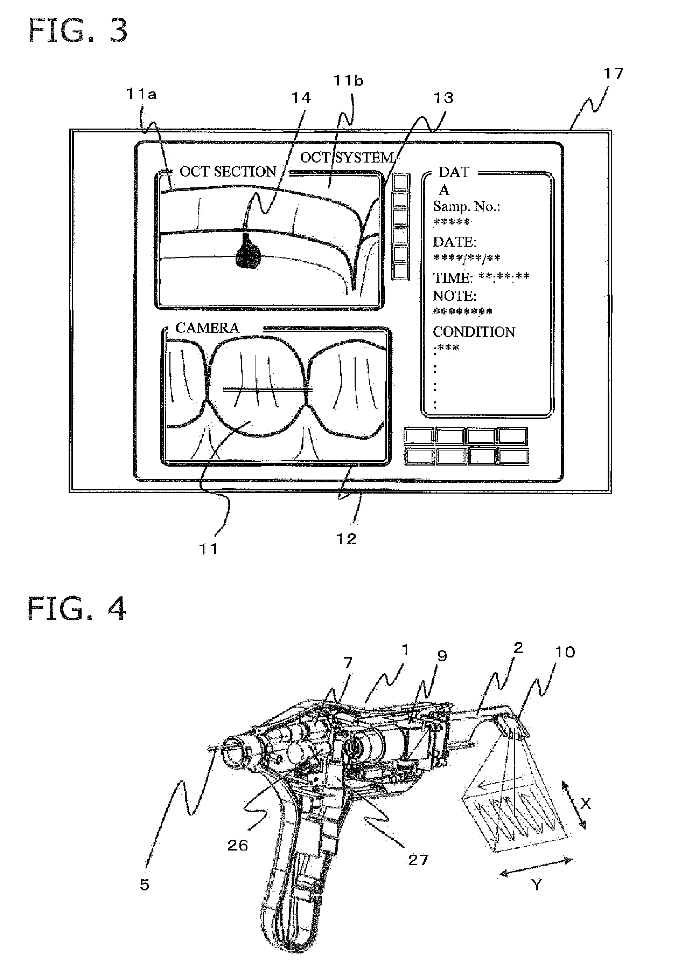

As shown in FIGS. 4 to 6, a collimating lens 7 that makes measurement-use near infrared light (with a wavelength of 1310 nm) into parallel light is provided inside the probe main body 1. The near infrared light emitted from this collimating lens 7 is scanned by an optical scanner 8 in a direction corresponding to the X axis direction (see FIG. 4, etc.), and then moved in a direction corresponding to the Y axis direction (which is perpendicular to the X axis direction), and then scanned again in the X axis direction. That is, with the optical tomography image acquisition device of this embodiment, scanning is performed in the same state as the scanning state used for image formation in a conventional picture-tube television set.

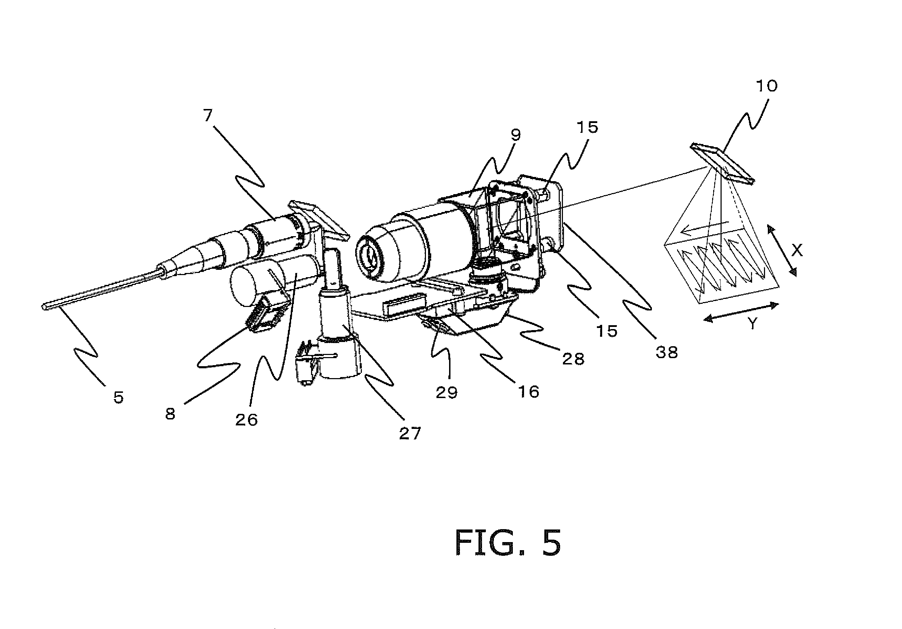

The scanned light is directed at the tooth 4 as shown in FIGS. 4 and 5 via a wavelength separating prism 9 and a reflecting mirror 10. At this point the light is scanned in the X axis direction of a tooth 11, as can be seen from the image of a display screen 12 shown in FIG. 3, and a tomographic image during scanning in this X axis direction is displayed on a display screen 13 above the other display screen shown in FIG. 3.

In FIG. 3, a cavity 14 that could not be discovered in the upper display screen 12 is displayed as a tomographic image on the display screen 13. For example, a cavity 14 that is only seen as a faint black smudge on the surface of the tooth 11 can be captured as a tomographic image, which reveals that there is a large downward opening, as shown on the display screen 13. This allows the caries of this tooth to be treated right away, which means that earlier treatment is possible.

In the next step, the position is moved slightly in the Y axis direction, and scanning is again performed in the X axis direction. The image at this point is displayed again on the display screen 13. Naturally, even if the tomographic image is not thus displayed right away on the display screen 13 after every scan in the X axis direction, the images can be checked later one at a time by manual operation by the dentist.

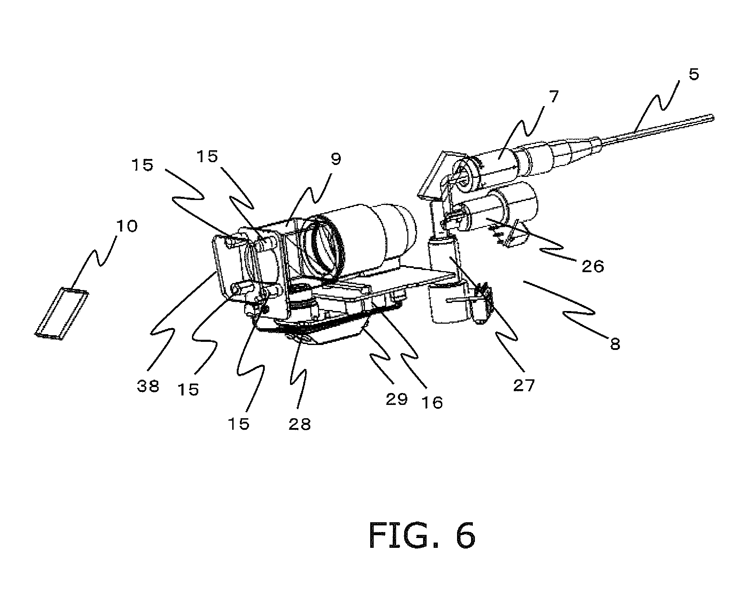

To produce these images, an illumination-use light emitting element 15 is disposed in front of the wavelength separating prism 9 as shown in FIGS. 5 and 6. The light from the light emitting element 15 shines on the tooth 11 via the reflecting mirror 10. This irradiation is reflected by the tooth, and light that is incident on the reflecting mirror 10 from the light input/output opening is again reflected by the reflecting mirror 10, goes to the wavelength separating prism 9, an internal reflecting mirror 28, and an internal reflecting mirror 29, and is detected as an image by a camera 16. This image is the image that is shown on the above-mentioned display screen 12.

In other words, the display screen 12 is displayed so that the user can recognize which tomographic image of the tooth 11 is currently being attempted to obtain. The user operates the probe main body 1 while looking at the image on the display screen 12, the result being that the scanning of near infrared light shown in FIGS. 4 and 5 is performed.

Thus, as shown in FIGS. 4 and 5, the scanned near infrared light moves straight through the wavelength separating prism 9, and passes through the collimating lens 7, after which it is returned through the cable 5 to the control box 6 (see FIG. 2). After undergoing image processing here, the image is displayed on the display screen 13 in FIG. 3. That is, FIG. 3 shows a display screen of a display component 17 of the control box 6.

The image processing in the control box 6 will now be described.

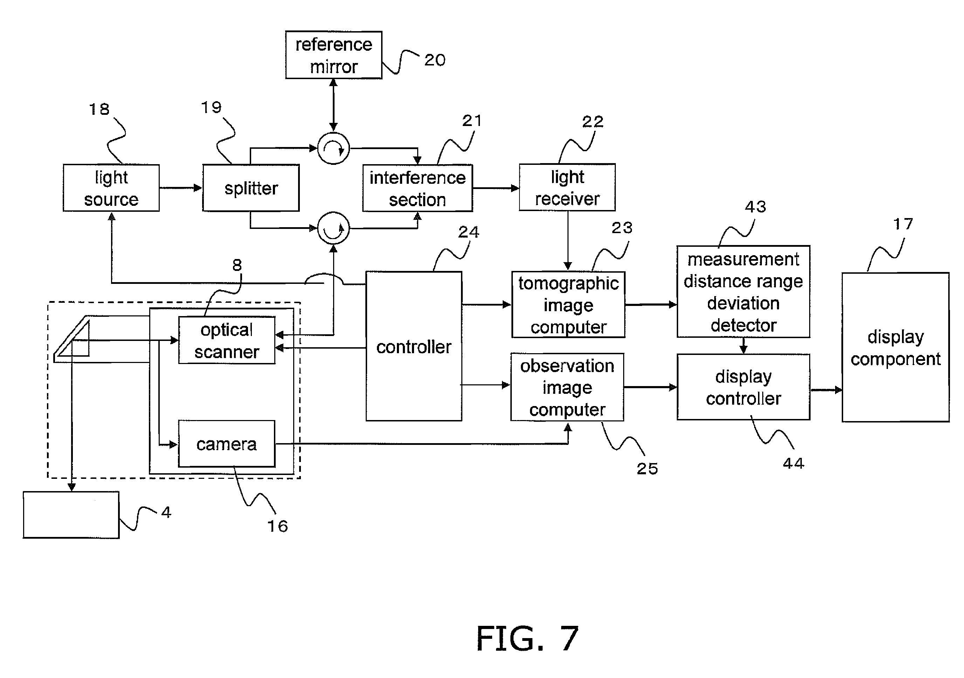

As shown in FIG. 7, the control box 6 of the optical tomography image acquisition device in this embodiment has a light source 18, a splitter 19, a reference mirror 20, an interference section 21, a light receiver 22, a tomographic image computer 23, a controller 24, an observation image computer 25, a measurement distance range deviation detector 43, a display controller (notification section, display controller) 44, and the display component 17.

The light source 18 is a wavelength sweep light source. The light emitted from the light source 18 is split up by the splitter 19, and part of it is supplied through the cable 5 to the optical scanner 8. Consequently, the above-mentioned tooth 4 is scanned in the X axis direction and the Y axis direction.

The rest of the light split up by the splitter 19 is reflected by the reference mirror 20 and supplied to the interference section 21. The interference section 21 produces interference light by causing interference between the light reflected by the reference mirror 20 and the light returned through the optical scanner 8 and the cable 5. This interference light is converted by the light receiver 22 into an electrical signal. This electrical signal is subjected to A/D conversion, after which the result is supplied to the tomographic image computer 23.

The tomographic image computer 23 performs FFT computation (fast Fourrier transform computation) on the A/D converted interference light, and acquires the surface shape of the tooth 4 (the measurement object) and tomographic image information about the same.

The controller 24 controls the observation image computer 25 and displays an observation image in real time on the display screen 12 (see FIG. 3). The controller 24 also controls the tomographic image computer 23 and displays a tomographic image on the display screen 13 (see FIG. 3).

The display range when a tomographic image is displayed on the display screen 13 will now be described.

The difference between the optical path length X from the above-mentioned splitter 19 to the interference section 21 via the reference mirror 20, and the optical path length Y from the splitter 19 to the interference section 21 going back to the distal end of the optical path of the light input/output portion 2 of the probe, that is, the light input/output portion 2, appears as the spectrum of interference light. The position at which the optical paths X = Y becomes the display start position S of the tomographic image displayed on the display screen 13. This display start position S will be discussed in detail at a later point.

The optical scanner 8 in this embodiment scans near infrared light from the collimating lens 7 in a first direction (the X direction in FIGS. 4 and 5) by means of the galvano scanner 26 shown in FIGS. 4, 5, and 6, and has galvano scanners 26 and 27 that scan in a second direction (the Y direction in FIGS. 4 and 5) perpendicular to the first direction.

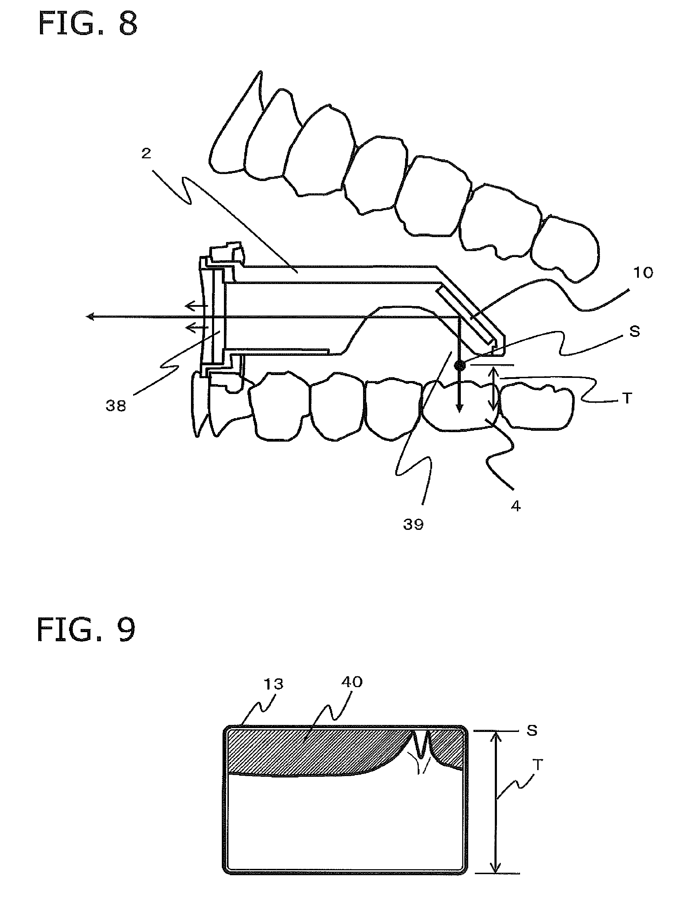

FIG. 8 shows the cross sectional structure when the light input/output portion 2 of the probe main body 1 has been inserted into the oral cavity. As discussed above, the light input/output portion 2 is provided in front of the probe main body 1. The reflecting mirror 10, which is a polarizing member, is provided at the distal end of the light input/output portion 2. A translucent protective cover 38 is provided so that the interior of the light input/output portion 2 is divided into front and back.

As discussed above, light that is incident from the probe main body 1 side passes through the protective cover 38, is polarized by the reflecting mirror 10 (a polarizing member), and irradiates the tooth 4 (the measurement object) through an opening 39. The light that irradiates the tooth 4 is reflected back through the opening 39 to the reflecting mirror 10, passes through the protective cover 38, and returns as measurement light to the probe main body 1 side.

A point S that becomes the above-mentioned display start position of the tomographic image is set between the tooth 4 and the opening 39 of the probe. The display start position S will now be defined.

First, the position at which the difference between the optical path length X from the above-mentioned splitter 19 to the interference section 21 via the reference mirror 20, and the optical path length Y from the splitter 19 to the interference section 21 via the light input/output portion 2 of the probe is equal becomes the display start position S.

In this embodiment, images of the surface of the tooth 4 and in the tomographic direction are acquired from the point of the display start position S.

The main characteristics in this embodiment will now be described. First, we will go back to FIG. 8 to describe the principle object of this embodiment.

The user can adjust the light irradiation angle and irradiation position of the probe manually, which makes the probe more convenient to use. On the other hand, if the probe is moved too close (beyond the measurement range) to the tooth that is the measurement object, aliasing may occur, in which measurement data outside the measurement range is displayed superposed with the other measurement data, so that a tomographic image of the measurement object cannot be displayed accurately.

To describe this in more specific terms through reference to FIG. 8, if the user moves the opening 39 of the light input/output portion 2 of the probe too close to the tooth 4 (beyond the display start position S), aliasing will end up occurring in which a tomographic image of the tooth 4 that is closer to the opening 39 than the display start position S is superposed with another tomographic image.

Next, FIG. 9 shows the display screen 13 when aliasing has occurred. An aliased part 40, which is indicated by hatching, occurs at the top of the display screen 13. This aliased part 40 is displayed in a state of being folded back in line symmetry and downward with respect to the horizontal line of the display start position S.

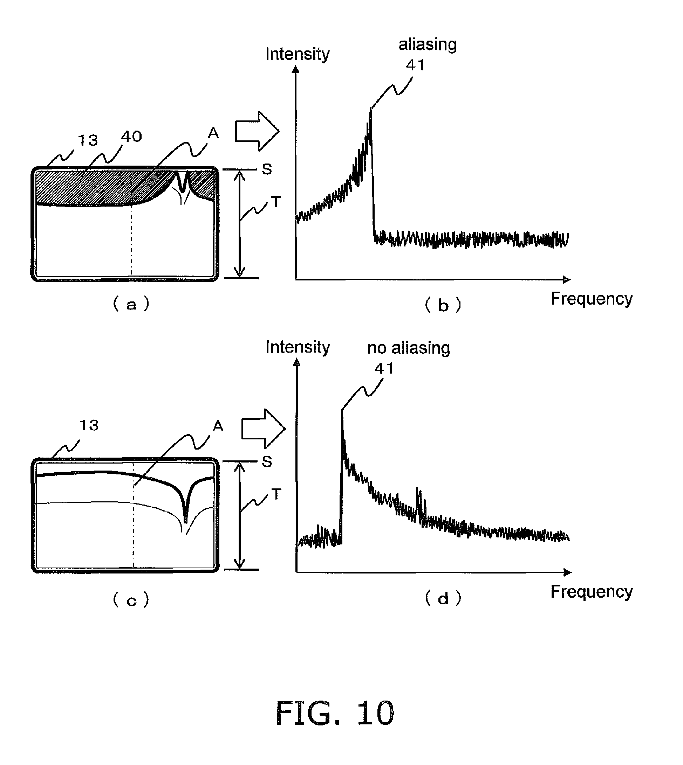

Next, the frequency spectrum of interference light in a state in which this aliased part 40 has been generated is shown in FIGS. 10a to 10 d .

FIG. 10b is a graph of the frequency spectrum at a location A at which the aliased part 40 has been generated in the display screen 13 of FIG 10a.

FIG. 10d is a graph of the frequency spectrum in a normal measurement state, in which no aliasing has occurred, on the display screen 13 in FIG. 10c.

A comparison of FIG. 10b and FIG. 10d reveals that the frequency spectrum in the normal measurement state shown in FIG. 10d has larger values on the high frequency side than on the low side of a frequency peak 41 corresponding to the surface position of the tooth 4. In contrast, the frequency spectrum when the aliased part 40 has been generated as shown in FIG. 10b has larger values on the low frequency side than on the high side of the frequency peak 41.

Specifically, with the optical tomography image acquisition device of this embodiment, peak detection is performed in the frequency spectrum of interference light, and the spectrum on the high frequency side is compared with the spectrum on the low frequency side over a specific frequency range centering on this peak detection position. Whether or not aliasing has occurred can be detected, and the aliased part can be identified, by detecting whether the values are larger on the high or low frequency side with respect to the peak detection position.

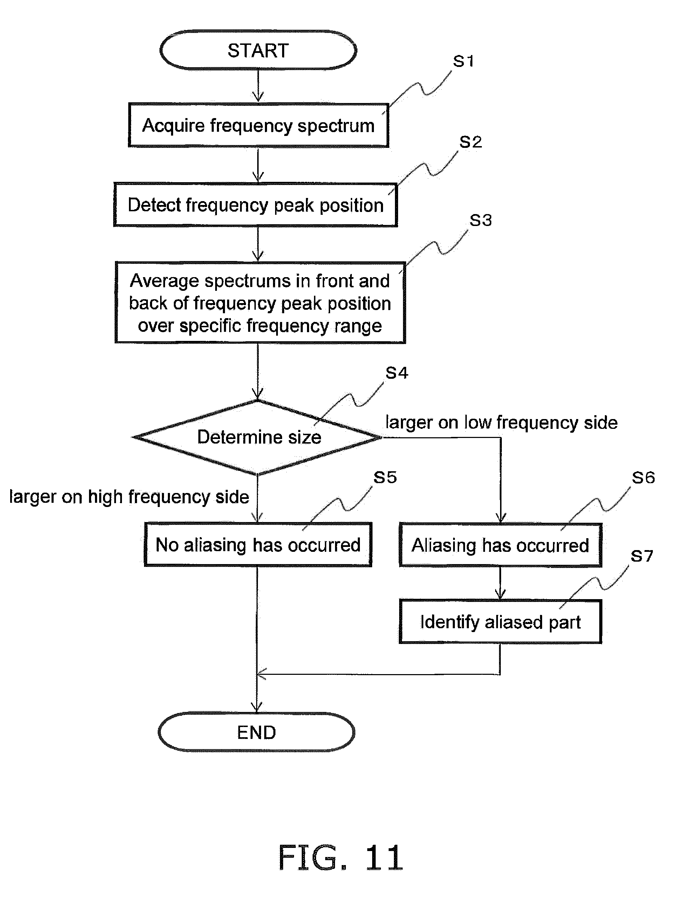

Next, FIG. 11 is a flowchart of detecting whether or not there is aliasing, and identifying the aliased part.

First, the frequency spectrum of interference light is acquired (S1).

Then, the frequency peak position is detected (S2).

Next, the spectrum on the high frequency side and the spectrum on the low frequency side are each averaged over a specific frequency range centering on the frequency peak position (S3).

The size of the averaged data is then determined (S4).

If the values are larger on the high frequency side, it is determined that "there is no aliasing" (S5).

On the other hand, if the values are larger on the low frequency side, it is determined that "there is aliasing" (S6), and the aliased part is identified (S7).

With the optical tomography image acquisition device in this embodiment, detection of whether or not aliasing has occurred and the identification of the aliased part are performed as above.

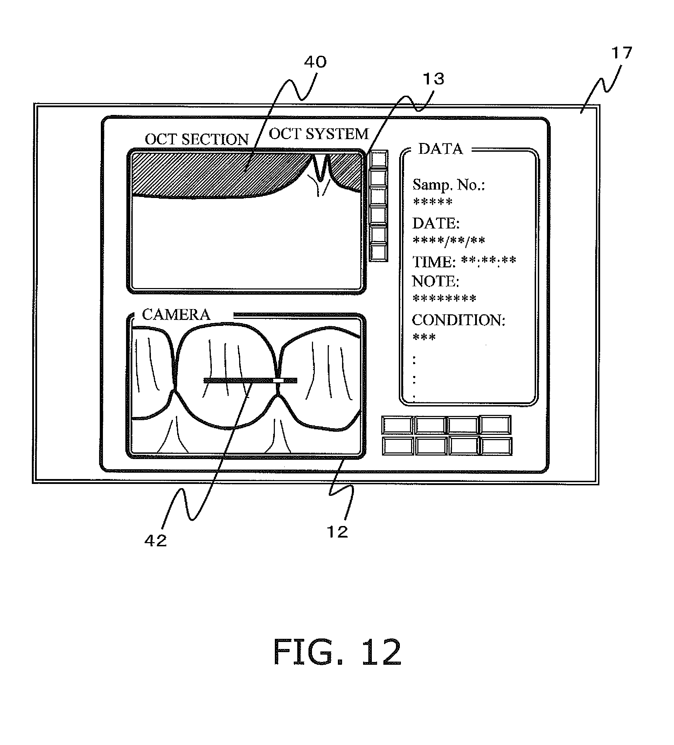

Next, FIG. 12 shows a display screen on the display component 17 when aliasing has been detected. As shown in FIG. 12, the aliased part 40 indicated by hatching occurs at the top of the display screen 13 displaying the tomographic image.

With the optical tomography image acquisition device in this embodiment, the aliased part 40 portion is displayed in color (such as red or orange) so that the user can recognize that the tomographic image includes this aliased part 40. Consequently, the user can easily recognize that the display includes aliasing merely by looking at the display screen 13 on the display component 17. As a result, the user can adjust so that a normal optical tomographic image is displayed by moving the position of the light input/output portion 2 of the probe main body 1 away from the tooth 4 or using another such measure.

A marker 42 indicating the measurement location is displayed in the middle of the display screen 12 displaying an observation image. The part of this marker 42 that is filled in black corresponds to the aliased part 40 of the tomographic image. On the display screen 12, the location on the marker 42 corresponding to the aliased part 40 and the location where no aliasing has occurred are displayed in different colors.

In some cases, a message or icon telling the user that aliasing has occurred may be displayed on the display component 17. When the occurrence of aliasing is thus conveyed in a more visual manner, the user can employ some measure such as moving the position of the light input/output portion 2 of the probe main body 1 a little farther away to adjust so that a tomographic image is obtained without any aliasing.

With the optical tomography image acquisition device in this embodiment, as discussed above, the measurement distance range deviation detector 43 uses the frequency spectrum of interference light from the tomographic image computer 23 to detect that the distance between the measurement object and the light input/output portion 2 of the probe is outside the measurement distance range when the measurement object is irradiated with light.

Also, when the display controller 44 displays this detection result on the display component 17, the user can look at the display component 17 and recognize that the current measurement data is outside the measurement range, with the light input/output portion 2 of the probe main body 1 being too close to the measurement object. Thus the user can adjust the position of the probe main body 1 in the oral cavity, and thereby readjust the position of the light input/output portion 2 to fall within the measurement range, so an accurate optical tomographic image can be displayed.

### Embodiment 2

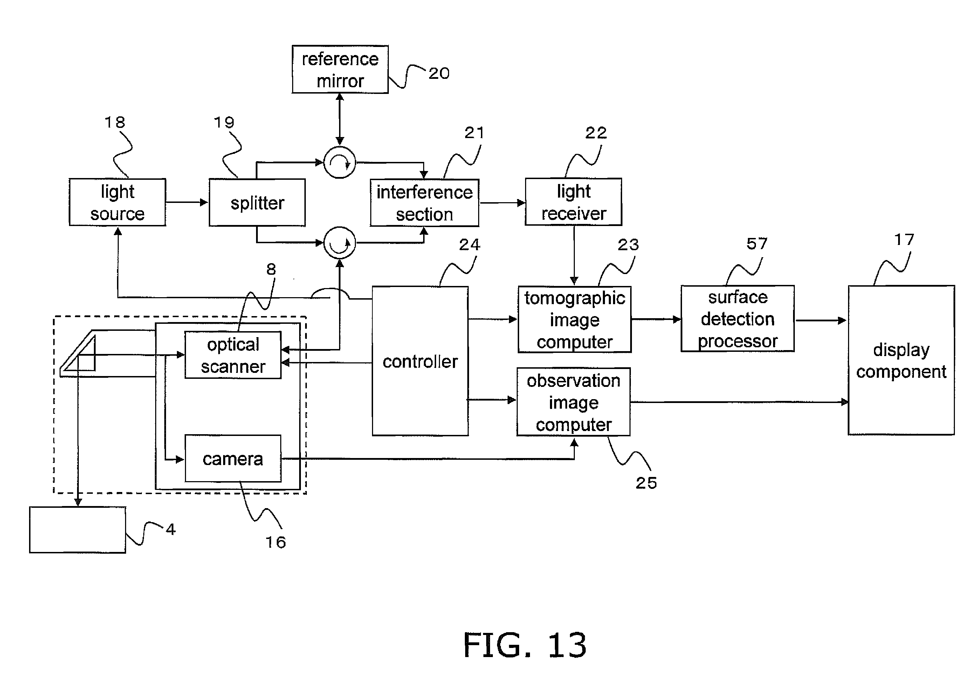

The optical tomography image acquisition device pertaining to another embodiment will now be described through reference to FIGS. 13 to 25. With the optical tomography image acquisition device in this embodiment, those components having the same function and shape as in Embodiment 1 above will for the sake of convenience be numbered the same, and will not be described again. More specifically, the optical tomography image acquisition device in this embodiment shares the components up to FIGS. 1 to 6 used in Embodiment 1 above.

The portions that differ from those in the optical tomography image acquisition device in Embodiment 1 will now be described.

Here, the image processing performed by the control box 6 of the optical tomography image acquisition device in this embodiment will be described.

As shown in FIG. 13, the control box 6 of the optical tomography image acquisition device in this embodiment has a light source 18, a splitter 19, a reference mirror 20, an interference section 21, a light receiver 22, a tomographic image computer 23, a controller 24, an observation image computer 25, a surface detection processor 57, and the display component 17.

Specifically, the optical tomography image acquisition device in this embodiment differs from that in Embodiment 1 above in that the surface detection processor 57 is provided instead of the measurement distance range deviation detector 43 and the display controller 44 as a component in the control box 6.

The method by which the surface detection processor 57 detects the surface of the tooth 4 will be discussed in detail at a later point.

Here, the protective cover 38 attached to the light input/output portion 2 of the probe main body 1 has the function of a waterproof cover, and keeps the rearward side waterproof with respect to the space on the front side of the light input/output portion 2.

Therefore, when the light input/output portion 2 side of the probe main body 1 is inserted into the oral cavity of a patient, since saliva, etc., of the patient penetrates through the opening 39 provided on the front side of the light input/output portion 2, the protective cover 38 has to be replaced, washed, etc. Thus, the light input/output portion 2 is configured to be removable from the probe main body 1. After being removed from the probe main body 1, the light input/output portion 2 can be washed and then used for the next patient.

However, washing so as to completely remove a patient's saliva, etc, adhering to the inner surface of the light input/output portion 2 makes the device less convenient for the user. Therefore, in this embodiment, a cover 38a is provided which covers the entire light input/output portion 2, including the opening 39, and can prevent the patient's saliva, etc., from penetrating through the opening 39. This makes the procedure more hygienic.

In this embodiment, with the above configuration, light that is incident from the probe main body 1 side passes through the protective cover 38, is polarized by the reflecting mirror 10 (a polarizing member), goes through the opening 39 and the cover 38a and is incident on the tooth 4 that is the measurement object. The light irradiated onto the tooth 4 is reflected, goes back through the cover 38a to the opening 39, passes through the reflecting mirror 10 and the protective cover 38, and comes back as measurement light to the probe main body 1 side.

A point S that becomes the above-mentioned display start position of the tomographic image is set between the tooth 4 and the opening 39 of the probe. The display start position S is defined the same as in Embodiment 1 above.

In this embodiment, images of the surface of the tooth 4 and in the tomographic direction are acquired from the point of the display start position S.

The main characteristics in this embodiment will now be described. First, we will go back to FIG. 3 to describe the principle object of this embodiment.

As discussed above, a tomographic image of the tooth 11 is displayed at the top of the display screen 13 in FIG. 3, and this tomographic image displays tomographic information by means of what is known as grayscale, or the density of white and black.

As to this tomographic information, information about the interior of the measurement object from the surface 11a of the tooth 11 (the measurement object) to the interior of the tooth 11 is important, whereas information about the so-called air layer 11b on the side closer to the probe than the surface 11a of the tooth 11 is essentially unnecessary. In view of this, there is a known display method in which the surface 11a of the tooth 11 is detected, information is separated into measurement object interior information and air layer information, and the tomographic information is made easier to recognize.

As to the method for detecting the surface 11a of the tooth 11, Japanese Laid-Open Patent Application 2007-225349 discloses a method in which the peak of the spectrum of an interference signal is detected for measurement light and reference light. With this method, however, the following problem is encountered.

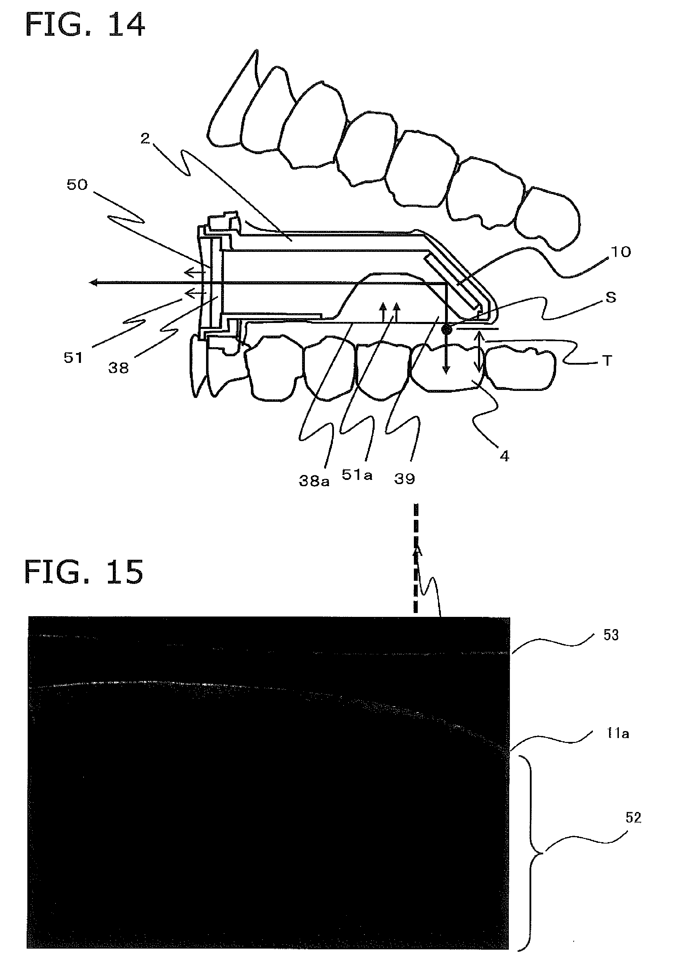

As shown in FIG. 14, light that is incident from the probe main body 1 side passes through the opening 39 and the cover 38a. Since the light does not completely pass through the interface of the cover 38a, some light is reflected to the rear, that is, rearward reflected light 51a is generated.

This rearward reflected light 51a mixes with the measurement light and appears as a peak in the spectrum of an interference signal in the interference section.

FIG. 15 shows a tomographic image when this rearward reflected light 51a has mixed with the measurement light. In the tomographic image shown in FIG. 15, interior information 52 about the tooth 11 is displayed below the surface 11a of the tooth 11. On the other hand, the interface 53 of the cover 38a is displayed above the surface 11a of the tooth 11.

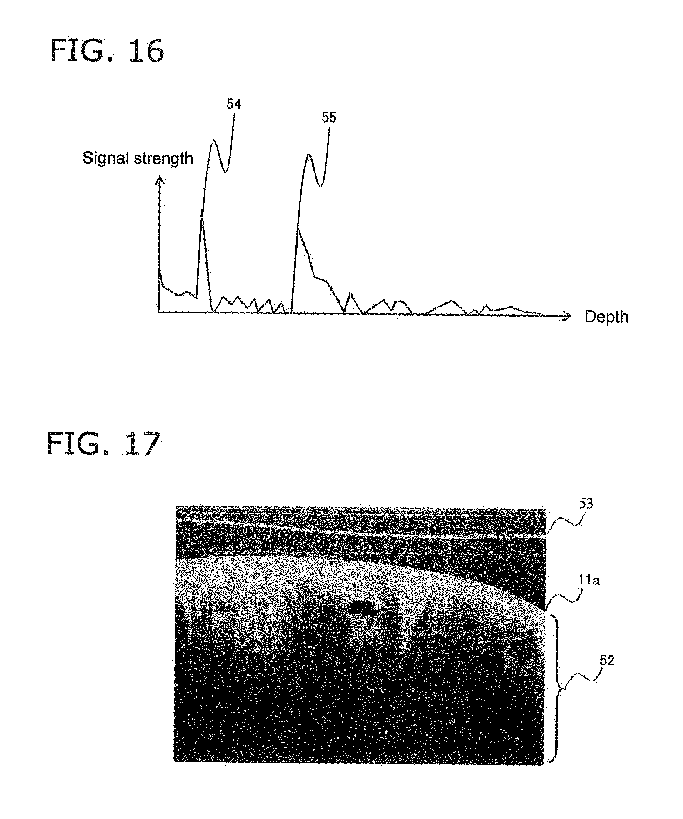

FIG. 16 shows the spectrum of interference light at the A line in FIG. 15. In this interference light spectrum, a first peak 54 produced by the interface 53 of the cover 38a and a second peak 55 produced by the surface 11a of the tooth 11 occur in that order starting from the low frequency side.

When two peaks occur in this way, if the surface 11a of the tooth 11 is simply detected as a peak in the spectrum of interference light, there is the risk that the interface 53 of the cover 3 8a will be mistaken for the surface 11a of the tooth 11. As a result, it may not be possible to display the tomographic image accurately.

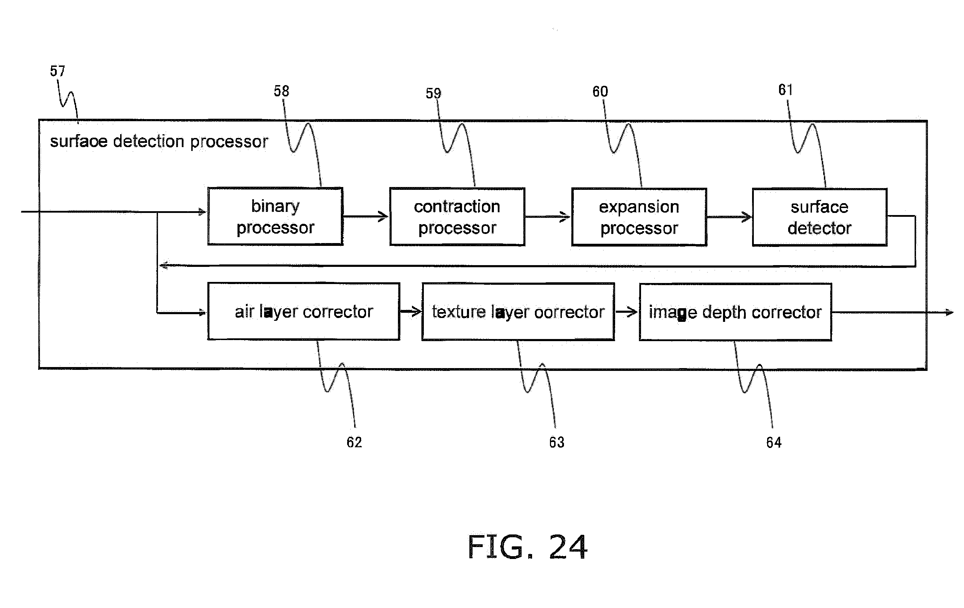

In view of this, with the optical tomography image acquisition device in this embodiment, as shown in FIG. 24, the surface detection processor 57 has, as internally produced function blocks, a binary processor 58, a contraction processor 59, an expansion processor 60, a surface detector 61, an air layer corrector (correction processor) 62, a texture layer corrector (correction processor) 63, and an image depth corrector 64.

As shown in FIG. 15, with tomographic information in which the interface 53 of the cover 38a is mixed, first the binary processor 58 performs a binary processing step in which the brightness value of display image data obtained from the tomographic image computer 23 shown in FIG. 13 is binarized into 0 and a normalized reference value with respect to a reference value. As a result, the tomographic image shown in FIG. 15 becomes a tomographic image exhibiting binarized, clear contrast as shown in FIG. 17.

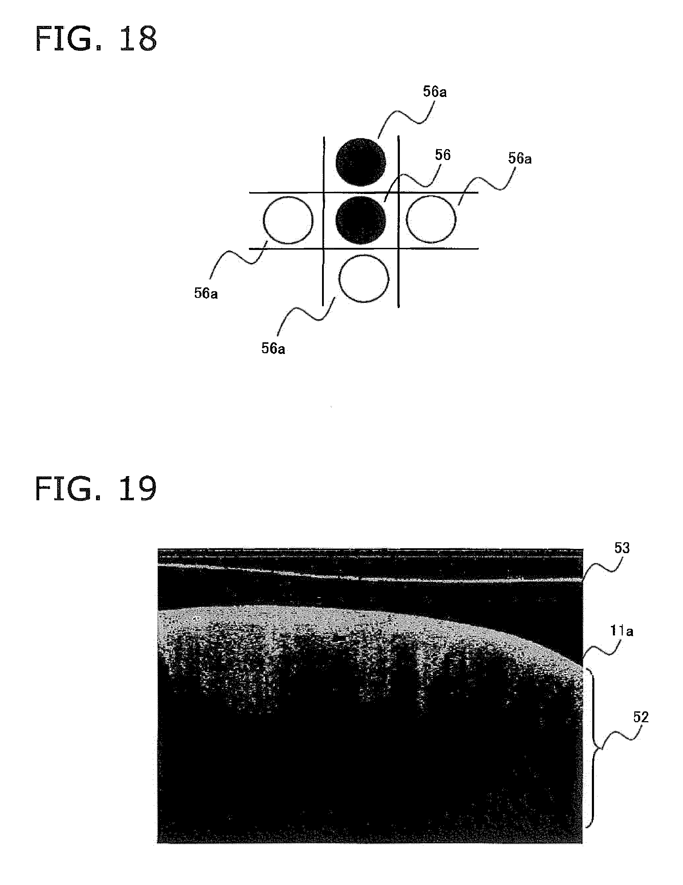

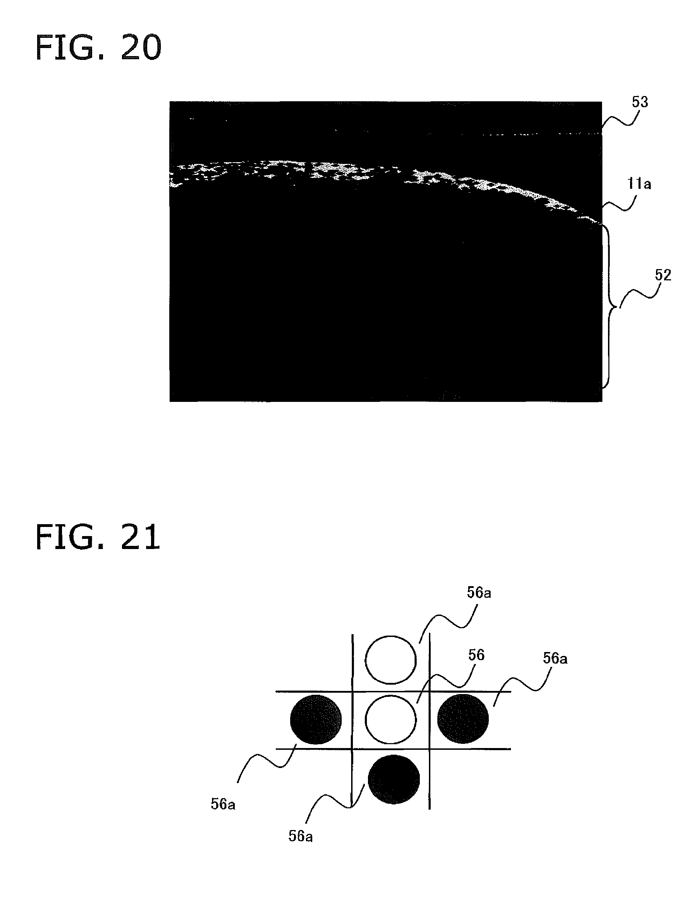

Next, as shown in FIG. 18, the contraction processor 59 performs a contraction step in which the brightness value for a specific pixel 56 is set to 0 if the brightness value is also 0 for at least one of the surrounding pixels 56a disposed above, below, to the left, and to the right of the specific pixel 56. This step is carried out from 1 to N times, the result being that the interface 53 steadily becomes thinner, as shown in FIGS. 19 and 20.

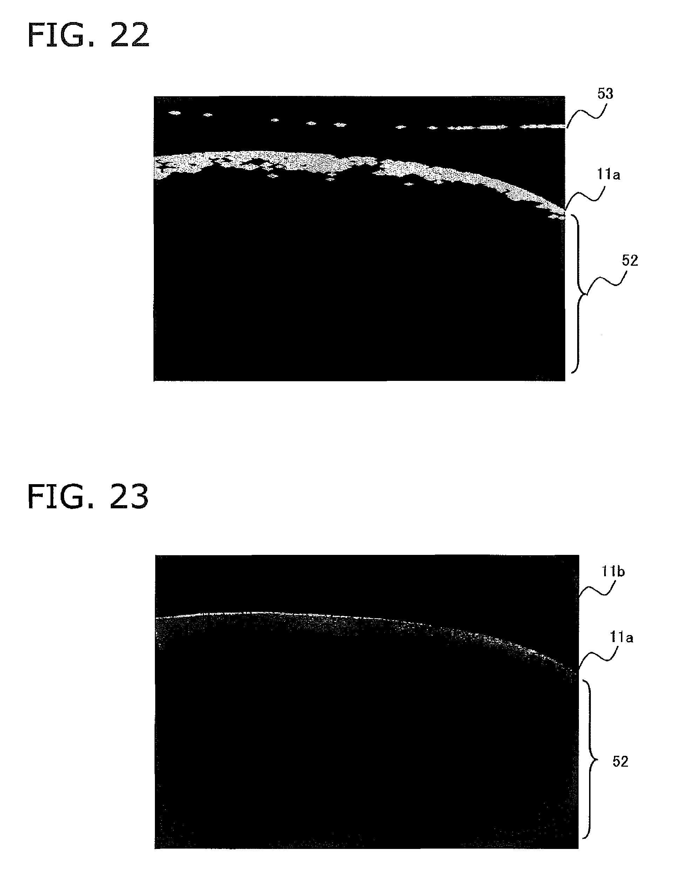

Next, the expansion processor 60 performs an expansion step in which the brightness value of the specific pixel 56 is set to a normalized reference value if the brightness value is also a normalized reference value for at least one of the surrounding pixels 56a disposed above, below, to the left, and to the right of the specific pixel 56, this being done for the tomographic image data that has undergone contraction processing N times, and for the brightness value of the specific pixel 56 as shown in FIG. 21. This step is carried out N times, the result being that the surface 11a of the tooth 11 shown in FIG. 22 is restored, while the interface 53 becomes disconnected, with no more connection as a line.

Next, the surface detector 61 performs a surface detection step in which the pixel serving as the normalized reference value among the tomographic information that has undergone expansion processing is used as a surface candidate point, this surface candidate point is compared to the original image data shown in FIG. 15, the sum of brightness values in the regions above and below the surface candidate point of the original image data is found, and if there is a large difference between the regions, a surface candidate line is used as the surface 11a of the tooth 11.

With the optical tomography image acquisition device in this embodiment, the surface 11a of the tooth 11 can be accurately detected by sequentially carrying out the above series of steps.

Next, after the surface detection of the tooth 11, the air layer corrector 62 and the texture layer corrector 63 classify the part above the surface as the air layer and the part below as tomographic information about the tooth 11 (measurement object internal information), and subject this tomographic information to display image correction. This allows the display image to be displayed in a way that is easier to see.

As to the processing in this correction step, processing may performed to set the brightness values to the same value using information about the air layer as non-measurement object data, and the dynamic range of brightness value of the measurement object internal information may be expanded.

As a result, as shown in FIG. 23, the interface 53 is eliminated, the brightness value of the air layer 11b (non-measurement object) has the same value, and the dynamic range of the brightness value of the tomographic image of the tooth 11 is expanded, which improves the contrast of the tomographic image of the tooth 11. Thus, the interior information 52 about the tooth 11 can be displayed more smoothly and with higher contrast.

With the optical tomography image acquisition device in this embodiment, the surface detection processor 57 is provided between the tomographic image computer 23 and the display component 17 as shown in the block diagram of FIG. 13 to perform the above processing.

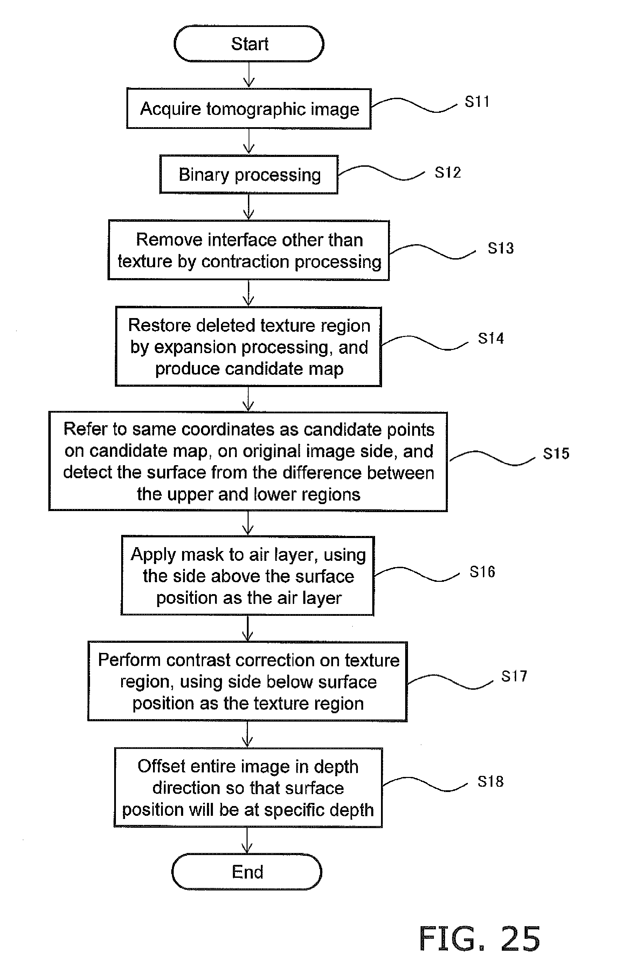

A diagram of the functional blocks produced in the interior of the surface detection processor 57 is shown in FIG. 24, and a flowchart of the processing performed by these functional blocks is shown in FIG. 25.

As shown in FIGS. 24 and 25, first tomographic information calculated by the tomographic image computer 23 is acquired (S11).

Then, the acquired tomographic information is binarized by the binary processor 58 (S12).

The contraction processor 59 then removes the interface within the non-measurement object other than the texture of a tooth (S 13).

The expansion processor 60 then restores information about texture that was eliminated by contraction processing (S 14).

Next, the surface detector 61 sets the pixel serving as the normalized reference value among the tomographic information as a surface candidate point, compares the original image data and the surface candidate point, finds the sum of brightness values in the regions above and below the surface candidate point of the original image data, and if there is a large difference between the regions, performs surface detection processing in which the surface candidate point is the surface of a tooth (S 15).

The air layer corrector 62 then compares the information obtained by surface detection to the tomographic information for the original image calculated by the tomographic image computer 23, and masks the tomographic image above the surface of the tooth obtained by surface detection of tomographic image about this original image (S16). The texture layer corrector 63 then corrects the contrast of the tomographic image below the surface of the tooth obtained by surface detection of tomographic image about this original image (S 17), allowing an accurate optical tomographic image to be displayed.

The image depth corrector 64 then displays the entire image offset in the depth direction so that the surface position in this tomographic image is at a specific depth (S 18). Specifically, the surface detection position is controlled so that its display is fixed at a specific position on the display screen of the display component 17. Consequently, even if there is movement due to the probe shaking while held in the user's hand, the tomographic image will be displayed at a fixed position with respect to the surface position of the tooth on the display screen of the display component 17, so the display screen will be easier to view.

### Embodiment 3

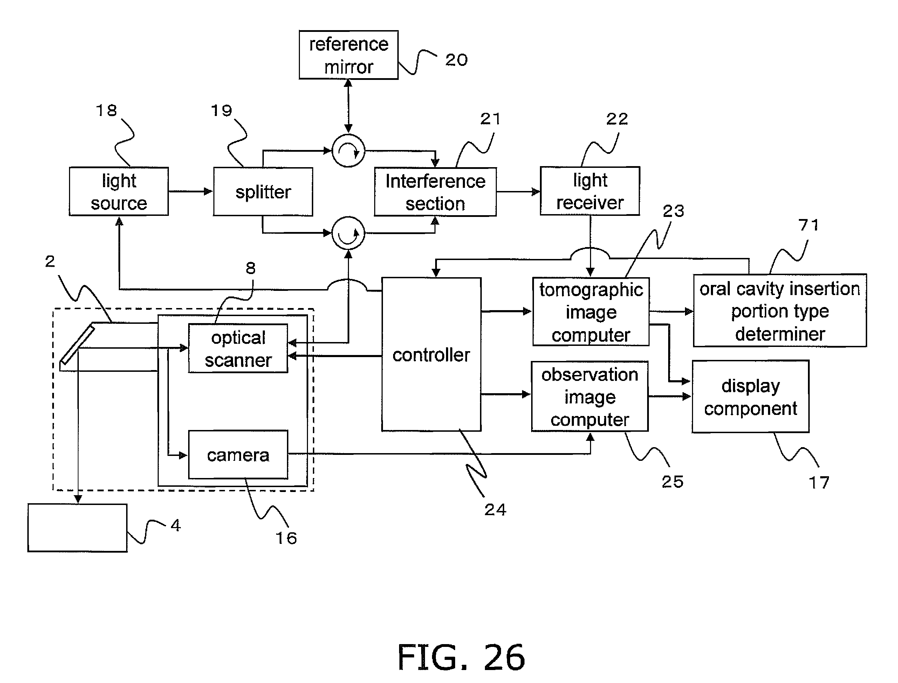

The optical tomography image acquisition device pertaining to yet another embodiment of the present invention will now be described through reference to FIGS. 26 to 31.

With the optical tomography image acquisition device in this embodiment, those components having the same function and shape as in Embodiment 1 above will for the sake of convenience be numbered the same, and will not be described again. More specifically, the optical tomography image acquisition device in this embodiment shares the components up to FIGS. 1 to 6 used in Embodiment 1 above.

The portions that differ from those in the optical tomography image acquisition device in Embodiment 1 will now be described.

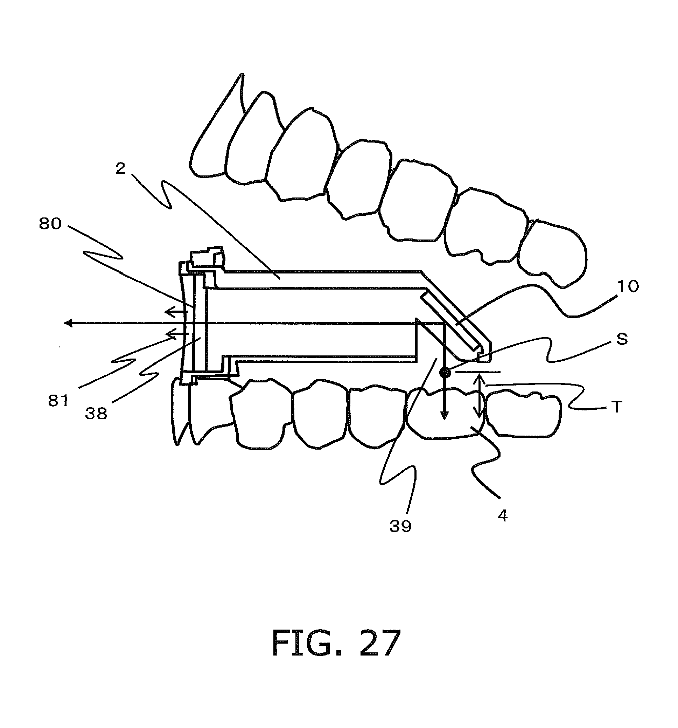

FIG. 27 shows the cross sectional structure of the light input/output portion 2 of the probe main body 1 (hereinafter referred to as the oral cavity insertion portion 2) in a state in which the oral cavity insertion portion 2 has been inserted into an oral cavity.

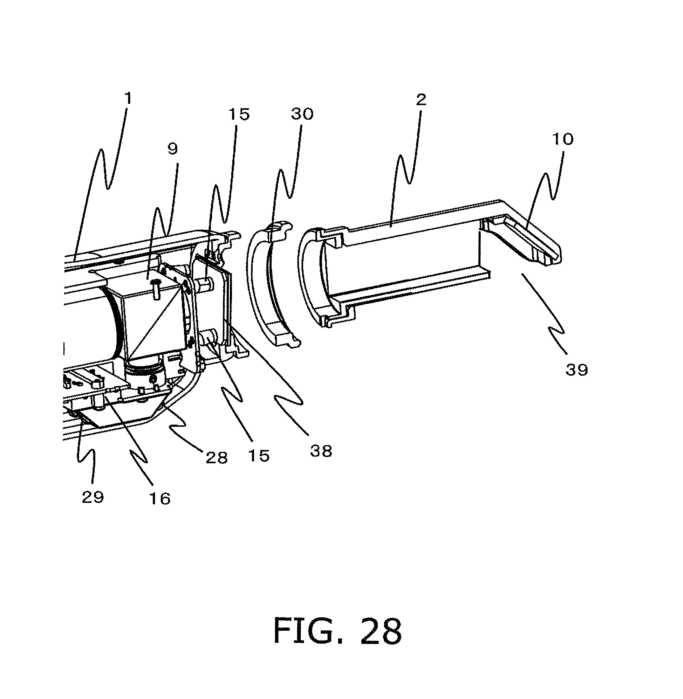

As discussed above, the oral cavity insertion portion 2 is provided in front of the probe main body 1. The reflecting mirror 10 (a polarizing member) is provided to the distal end of the oral cavity insertion portion 2. As shown in FIG. 28, the translucent protective cover 38 is provided so that the oral cavity insertion portion 2 and the probe main body 1 are divided into front and back.

Returning to FIG. 27, as discussed above, light that is incident from the probe main body 1 side passes through the protective cover 38, is polarized by the reflecting mirror 10 (a polarizing member), and irradiates the tooth 4 (the measurement object) through the opening 39. The light that irradiates the tooth 4 is reflected back through the opening 39 to the reflecting mirror 10, passes through the protective cover 38, and returns as measurement light to the probe main body 1 side.

A point S that becomes the above-mentioned display start position of the tomographic image is set between the tooth 4 and the opening 39 of the probe. The display start position S is defined the same as in Embodiment 1 above.

With the optical tomography image acquisition device in this embodiment, images of the surface of the tooth 4 and in the tomographic direction are acquired from the point of the display start position S.

The main characteristics in this embodiment will now be described.

As discussed above, the size and shape of the oral cavity insertion portion 2 of the probe in this embodiment will vary from one patient to the next, such as between adults and children. Therefore, the oral cavity insertion portion 2 is removably mounted to the probe main body 1.

In this embodiment, the following configuration is employed so that the type of oral cavity insertion portion 2 mounted to the probe main body 1 can be determined automatically.

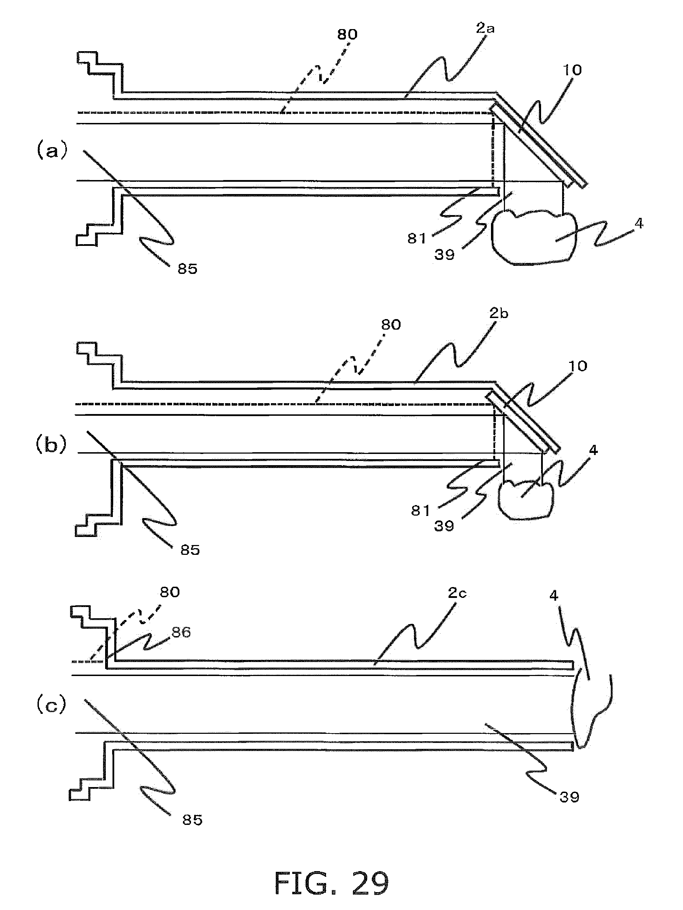

FIGS. 29a to 29c are diagrams illustrating the method for determining the type of oral cavity insertion portion 2.

FIG. 29a shows a state in which an oral cavity insertion portion 2a used for observing the back teeth of an adult is installed in a dental-use optical tomography image acquisition device.

FIG. 29b shows a state in which an oral cavity insertion portion 2b used for observing the back teeth of a child is installed.

With the oral cavity insertion portions 2a and 2b used observing back teeth, the reflecting mirror 10 is provided at the distal end thereof so that irradiation light and measurement light can be polarized to make the back teeth easier to observe. Thus, this is a configuration with which a tomographic image of back teeth or the like is easy to acquire.

FIG. 29c shows a state in which an oral cavity insertion portion 2c used for observing the front teeth is installed. The oral cavity insertion portion 2c irradiates the front teeth with light and takes in measurement light directly, without polarizing the light first, so that the front teeth can be observed more easily.

The method for determining the type of oral cavity insertion portions 2a and 2b used for observing back teeth will now be described through reference to FIGS. 29a and 29b.

First, the irradiation direction of the optical scanner 8 is controlled to be a type determination optical path 80 of the oral cavity insertion portions 2a and 2b, and an irradiation wall 81 provided to the rear of the opening 39 of the oral cavity insertion portions 2a and 2b is irradiated. The light that irradiates the irradiation wall 81 then becomes reflected light and returns to the type determination optical path 80, goes through the optical scanner 8, and is taken in as measurement light by the interference section 21. The tomographic image computer 23 finds the frequency spectrum of this interference light.

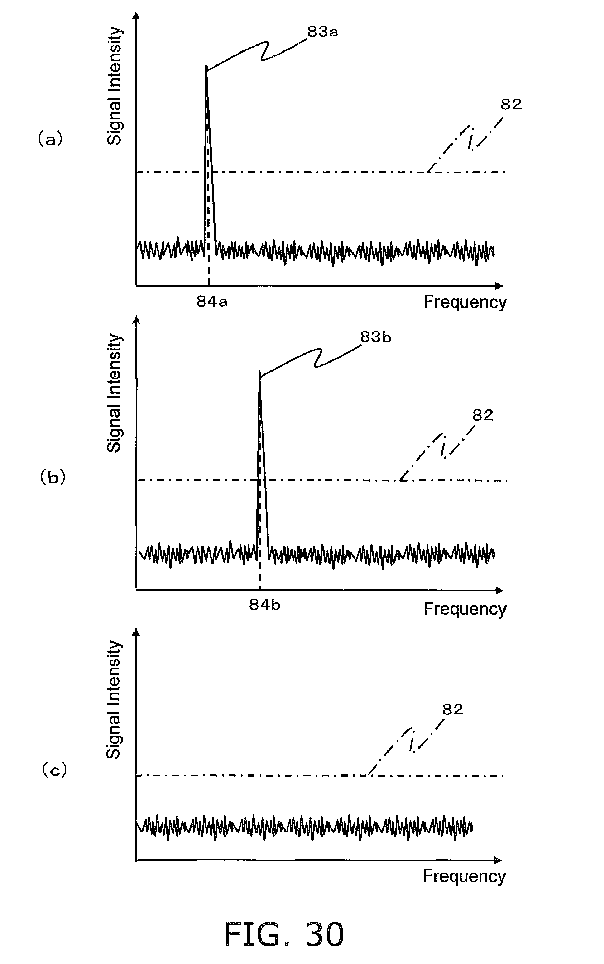

FIGS. 30a to 30c show the frequency spectrum.

An oral cavity insertion portion type determiner 71 calculates frequency peaks 83a and 83b that exceed a specific threshold 82 of the obtained frequency spectrum, and determines the type of oral cavity insertion portions 2a and 2b on the basis of the frequencies 84a and 84b of these frequency peaks 83a and 83b.

FIG. 30a shows the frequency spectrum in a state in which the adult-use oral cavity insertion portion 2a shown in FIG. 29a is installed. FIG. 30b shows the frequency spectrum in a state in which the child-use oral cavity insertion portion 2b shown in FIG. 29b is installed.

With the optical tomography image acquisition device in this embodiment, the adult-use oral cavity insertion portion 2a shown in FIG. 29a and the child-use oral cavity insertion portion 2b shown in FIG. 29b have the same length (the length in the left and right direction in the drawings, but have different heights (the length in the up and down direction in the drawings).

Consequently, the difference in height between the oral cavity insertion portions 2a and 2b appears as a difference in the path length of the measurement light. Therefore, in a frequency spectrum for recognizing the path length of the measurement light by frequency, this difference appears as a difference in the frequency of the frequency peaks of the above-mentioned frequency spectrum.

More specifically, with the child-use oral cavity insertion portion 2b having a shorter path length, as shown in FIG. 30b, the frequency peak has a frequency 84b. The frequencies corresponding to these frequency peak are such that in a state in which the adult-use oral cavity insertion portion 2a whose path length is longer than that of the child-use oral cavity insertion portion 2b is installed, the frequency 84a results, which is lower than the frequency 84b, as shown in FIG. 30a, so there is a difference between the two.

In this embodiment, because of the above, the oral cavity insertion portion type determiner 71 compares the frequencies corresponding to the frequency peak and thereby determines the type of the oral cavity insertion portions 2a and 2b.

Next, the method for determining the type of the oral cavity insertion portion 2c used for observing front teeth shown in FIG. 29c will be described.

In this case, the irradiation direction of the optical scanner 8 is controlled to be the type determination optical path 80 of the oral cavity insertion portion 2c, and an irradiation wall 86 provided around a rear opening 85 of the oral cavity insertion portion 2c is irradiated.

The light that irradiates the irradiation wall 86 then becomes reflected light and returns to the type determination optical path 80, goes through the optical scanner 8, and is taken in as measurement light by the interference section 21. The tomographic image computer 23 finds the frequency spectrum of this interference light.

FIG. 30c shows the frequency spectrum here. There is no frequency peak in the frequency spectrum shown in FIG. 30c. This is because the position of the irradiation wall 86 is outside the interference range. This interference range is determined by the coherence length of light emitted from the light source 18. The interference range in this embodiment is set to a range of ± 10 mm around the display start position S in FIG. 27.

That is, in the state shown in FIG. 29c, in which the oral cavity insertion portion 2c for observing front teeth is installed, the type of the oral cavity insertion portion 2c can be determined by detecting that the path length of measurement light is outside the interference range during determination of the type of oral cavity insertion portion 2c.

Specifically, with the optical tomography image acquisition device in this embodiment, the optical scanner 8 of the probe performs control so that the oral cavity insertion portions 2a to 2c are irradiated with light and this reflected light is taken in as measurement light. The type of the oral cavity insertion portions 2a to 2c can be determined on the basis of the computation result of the tomographic image computer 23 for the interference light obtained from this measurement light. Thus, it is possible to determine the type of the oral cavity insertion portions 2a to 2c by using the tomographic image acquisition function with which the apparatus is already equipped, without providing a switch or other such electrical or mechanical detector. This allows the probe to be more compact.

Next, the timing at which the type of these oral cavity insertion portions 2a to 2c is determined will be described.

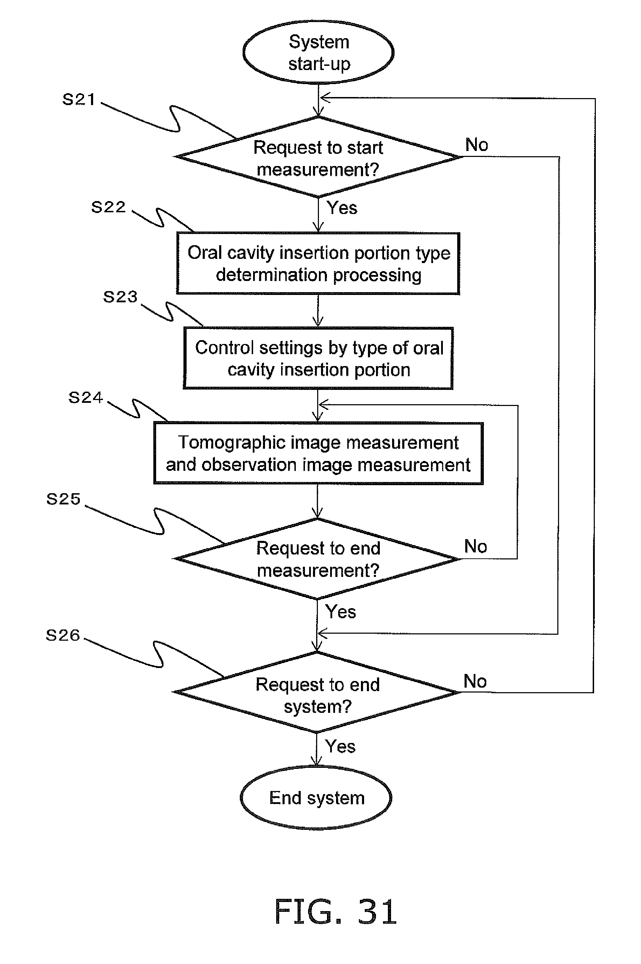

FIG. 31 is a flowchart of the timing at which the type of oral cavity insertion portions 2a to 2c is determined.

First, after the system has been started up, it is decided whether or not there is a measurement start request (S21). If there is a measurement start request, the above-mentioned determination of the type of the oral cavity insertion portions 2a to 2c is carried out (S22), and the flow proceeds to step S23. On the other hand, if there is no measurement start request, the flow proceeds to step S26.

Next, a setting is made according to the oral cavity insertion portions 2a to 2c determined in step S22 (S23).

Then, tomographic image measurement and observation image measurement of the measurement object are performed (S24).

It is then confirmed whether or not there has been a measurement end request (S25).

If there has been a measurement end request here, the flow proceeds to step S26, and it is confirmed whether or not there has not been a system end request (S26). On the other hand, if there has been no system end request, the flow returns to step S24.

It is then confirmed whether or not there has been system end request (S26). If there has been a system end request, the system is ended. If there has been no system end request, however, the flow returns to step S21.

In this embodiment, as discussed above, the type of oral cavity insertion portion 2 is determined every time measurement is performed, and as a result, the proper measurement can be performed according to the type of oral cavity insertion portion 2 during measurement even if the user replaces the oral cavity insertion portion 2 when the power is off or when measurement is not in progress.

Furthermore, by providing a locking mechanism or the like that disables the replacement of the oral cavity insertion portion 2 when the power is on, it is possible to determine the type of the oral cavity insertion portion 2 only when the power has been switched on, which allows the design of the system to be simplified.

### Other Embodiments

Embodiments of the present invention were described above, but the present invention is not limited to or by these embodiments, and various modifications are possible without departing from the gist of the invention.

### (A)

In the above embodiments, an example was given in which, if it is detected that the distance between the measurement object and the light input/output portion 2 of the probe is outside the measurement distance range, the display controller 44 puts a display on the display component 17 that allows the user to recognize that the measurement distance range has been exceeded, and tells the user that the correct tomographic image has not been displayed. However, the present invention is not limited to this.

For example, a notification section may be provided separately from the display controller, and it may be used to tell the user that the correct tomographic image has not been displayed.

That is, in the above embodiments, the display controller 44 comprised the function of the notification section and the display controller of the present invention, but the optical tomography image acquisition device may have a configuration that functions as the notification section of the present invention, separately from the display controller.

### (B)

In the above embodiments, an example was described in which the present invention was applied to an optical tomography image acquisition device comprising the display component 17, but the present invention is not limited to this. For example, the present invention may be applied to an optical tomography image acquisition device that has no display component. In this case, the means for notifying the user with the notification section can be notification by light at the probe distal end, notification by sound, displaying a notification on an externally connected notification section, or the like.

### (C)

In the above embodiments, an example was described in which the notification means for allowing the user to recognize that aliasing had occurred was to perform display control in which the aliased part 40 was displayed in color on the display component 17, but the present invention is not limited to this. For example, instead of coloring the aliased part, some other notification means may be employed, such as giving an audible alarm, or emitting light from the distal end portion of the probe, or flashing the display of the aliased part.

### INDUSTRIAL APPLICABILITY

With the optical tomography image acquisition device of the present invention, as discussed above, the user can recognize that the current position of the light input/output portion of the probe is outside the measurement range with respect to the measurement object, so the user can readjust the position of the light input/output portion of the probe to be within the measurement range right away, and an accurate optical tomographic image can be displayed. Because of this, the present invention is expected to find wide application as a dental-use optical tomography image acquisition device, for example.

### REFERENCE SIGNS LIST

- 1: probe main body
- 2: light input/output portion (oral cavity insertion portion)
- 2a: oral cavity insertion portion (for observing back teeth in adults)
- 2b: oral cavity insertion portion (for observing back teeth in children)
- 2c: oral cavity insertion portion (for observing front teeth)
- 3: oral cavity
- 4: tooth
- 5: cable
- 6: control box
- 7: collimating lens
- 8: optical scanner
- 9: wavelength separating prism
- 10: reflecting mirror
- 11: tooth
- 11a: surface
- 11b: air layer
- 12: display screen
- 13: display screen
- 14: cavity
- 15: light emitting element
- 16: camera
- 17: display component
- 18: light source
- 19: splitter
- 20: reference mirror
- 21: interference section
- 22: light receiver
- 23: tomographic image computer
- 24: controller
- 25: observation image computer
- 26: galvano scanner
- 27: galvano scanner
- 28: internal reflecting mirror
- 29: internal reflecting mirror
- 30: light input/output portion mounting screw
- 38: protective cover
- 38a: cover
- 39: opening
- 40: aliased part
- 41: frequency peak
- 42: marker
- 43: measurement distance range deviation detector
- 44: display controller (notification section, display controller)
- 51a: rearward reflected light
- 52: interior information
- 53: interface
- 54: first peak
- 55: second peak
- 56: specific pixel
- 56a: surrounding pixels
- 57: surface detection processor
- 58: binary processor
- 59: contraction processor
- 60: expansion processor
- 61: surface detector
- 62: air layer corrector (correction processor)
- 63: texture layer corrector (correction processor)
- 64: image depth corrector
- 71: oral cavity insertion portion type determiner
- 80: type determination optical path
- 81: irradiation wall
- 82: threshold
- 83a, 83b,: frequency peak
- 84a, 84b: frequency
- 85: rear opening
- 86: irradiation wall

## Claims

1. An optical tomography image acquisition device, comprising:
a light source;
a splitter configured to split light emitted from the light source into a first direction and a second direction;
a probe has a light input/output portion configured to direct first light split off in the first direction toward a measurement object, and take in reflected light from the measurement object as measurement light;
a reference mirror configured to perform path correction on second light split off in the second direction at the splitter;
an interference section configured to produce interference light by causing interference between the second light that has undergone path correction at the reference mirror and the first light taken in as measurement light to the probe;
a tomographic image computer configured to produce tomographic image information about a measurement object by computational processing of interference light produced at the interference section;
a measurement distance range deviation detector configured to detect whether or not the distance between the measurement object and the light input/output portion of the probe is outside of a measurement distance range on the basis of the computation result of the tomographic image computer; and
a notification section configured to notify of the detection result by the measurement distance range deviation detector.

2. The optical tomography image acquisition device according to Claim 1,
wherein the measurement distance range deviation detector configured to perform peak detection of the frequency spectrum of interference light that is the computation result of the tomographic image computer, compare the sizes of the spectrum on the high frequency side and the spectrum on the low frequency side in a specific frequency range with respect to the peak detection position, detect whether or not aliasing in which a tomographic image outside the measurement distance range is displayed superposed with a normal tomographic image has occurred, and thereby detect when the distance between the measurement object and the light input/output portion of the probe is outside the measurement distance range.

3. The optical tomography image acquisition device according to Claim 1 or 2, further comprising:
a display component configured to display the computation result of the tomographic image computer and the tomographic image information, and also display the detection result of the measurement distance range deviation detector; and
a display controller configured to control the display component.

4. The optical tomography image acquisition device according to Claim 3,
wherein the display controller controls the display component so as to change the display of the color of the tomographic image information that is outside the measurement distance range.

5. The optical tomography image acquisition device according to Claim 3 or 4,
wherein the display controller controls the display component so as to display a place outside the measurement distance range as an observation image.

6. An optical tomography image acquisition device, comprising:
a light source;
a splitter configured to split light emitted from the light source into a first direction and a second direction;
a probe has a light input/output portion configured to direct first light split off in the first direction toward a measurement object, and take in reflected light from the measurement object as measurement light;
a reference mirror configured to perform path correction on second light split off in the second direction at the splitter;
an interference section configured to produce interference light by causing interference between the second light that has undergone path correction at the reference mirror and the first light taken in as measurement light to the probe;
a tomographic image computer configured to produce tomographic image information about a measurement object by computational processing of interference light produced at the interference section; and
a surface detection processor configured to output the computation result of the tomographic image computer and that has a binary processor that binarizes a brightness value for display image data obtained from the tomographic image computer into 0 and a normalized reference value with respect to a reference value, a contraction processor that sets the brightness value for a specific pixel to 0 when the brightness value is 0 for at least one pixel out of the surrounding pixels of a specific pixel of binarized display image data, an expansion processor that sets the brightness value of a specific pixel to a normalized reference value when the brightness value of at least one pixel out of the surrounding pixels of a specific pixel of display image data that has undergone contraction processing is a normalized reference value, a surface detector that detects the surface of a measurement object from display image data that undergone expansion processing, and a correction processor that compares display image data obtained from the tomographic image computer and the surface of a measurement object detected by the surface detector and performs display image correction.

7. The optical tomography image acquisition device according to Claim 6,
further comprising a display component configured to receive tomographic image information outputted from the surface detection processor.

8. The optical tomography image acquisition device according to Claim 7,
further comprising an image depth corrector configured to offset the surface position of the measurement object in the depth direction of the measurement object so that the surface position will be at a specific location on the display screen of the display component.

9. The optical tomography image acquisition device according to any of Claims 6 to 8,
wherein the correction processor configured to perform processing to set the brightness value of data for a non-measurement object to the same value.

10. The optical tomography image acquisition device according to any of Claims 6 to 9,
wherein the correction processor configured to perform processing to expand the dynamic range of the brightness value for data of a measurement object.

11. An optical tomography image acquisition device, comprising:
a light source;
a splitter configured to split light emitted from the light source into a first direction and a second direction;
a probe that has a probe main body, an optical scanner that directs first light split off in the first direction toward a measurement object while varying the irradiation direction and that is provided inside the probe main body, and an oral cavity insertion portion that is provided with a light input/output portion that takes in reflected light from the measurement object as measurement light and that is removably attached to the probe main body;
a reference mirror configured to perform path correction on second light split off in the second direction at the splitter;
an interference section configured to produce interference light by causing interference between the second light that has undergone path correction at the reference mirror and the first light taken in as measurement light to the probe;
a tomographic image computer configured to produce tomographic image information about a measurement object by computational processing of interference light produced at the interference section; and
an oral cavity insertion portion type determiner configured to determine the type of oral cavity insertion portion on the basis of the computation result of the tomographic image computer.

12. The optical tomography image acquisition device according to Claim 11,
further comprising a display component configured to receive tomographic image information outputted from the tomographic image computer.

13. The optical tomography image acquisition device according to Claim 11 or 12,
wherein the oral cavity insertion portion type determiner determines the type of oral cavity insertion portion when the power is switched on.

14. The optical tomography image acquisition device according to any of Claims 11 to 13,
wherein the oral cavity insertion portion type determiner determines the type of oral cavity insertion portion when measurement begins.

15. The optical tomography image acquisition device according to any of Claims 11 to 14,
wherein the oral cavity insertion portion has an irradiation wall that emits light for the type determination.

16. The optical tomography image acquisition device according to any of Claims 11 to 15,
wherein the oral cavity insertion portion type determiner determines the type of the oral cavity insertion portion on the basis of the path length of measurement light during type determination.

17. The optical tomography image acquisition device according to any of Claims 11 to 15,
wherein the oral cavity insertion portion type determiner determines the type of the oral cavity insertion portion by detecting that the path length of measurement light during type determination is outside an interference range.
